# EUROPEAN PATENT APPLICATION

(11) **EP 1 832 264 A1**
(43) Date of publication of application: **12.09.2007**
(21) Application number: 07250976.3
(22) Date of filing: 08.03.2007
(51) Int. Cl.: A61F 13/15

(54) **Refastenable tab for disposable pant and methods for making same**

(30) Priority: 08.03.2006 US 780404 P
(71) Applicant: Curt G. Joa, Inc., Sheboygan Falls, WI 53085-0903 (US)
(72) Inventor: Andrews, Robert E., Sheboygan Wuscibsub 53085 (US)
(74) Representative: Dee, Ian Mark

(57) **Abstract**

A pants type diaper is disclosed having a detachable coupling point that allows a user to pull on the coupling point and re-fasten the coupling point around a waist of the user, to cause the pants type diaper to be snugly disposed against the waist of the user so that the diaper can be comfortably worn.

## Description

### Related Application

This application claims the benefit of United States Provisional Patent Application Serial No. 60/780,404, filed 8 March 2006, and entitled "Refastenable Tab for Disposable Pant and Methods for Making Same"

### Background of the Invention

The present invention relates to disposable undergarments and, more specifically, to methods and apparatuses for forming disposable undergarments, and particularly to refastenable tabs that are preferably expandable.

The disposable garments are typically produced in a laid-flat blank format, with the blanks forming a continuous web of material. The final form of the diaper is achieved by folding the blanks in half, either before or after they are cut from the web of material. The blanks must also be sealed along the side edges to form the final diaper shape in a pants type diaper. The present invention relates to either forming a coupling about a seam of a pants type diaper, or for coupling about a waist for non-pants type diapers.

Generally, disposable undergarments such as pants-type diapers are made up of two nonwoven layers of material with elastic strands of material placed between the two nonwoven layers of material thus creating an elastic web laminate. The layers of material are continuous sheets of material that are eventually cut into individual undergarment lengths. The elastic strands may be arranged and cut so that specific areas of the undergarment are free of elastic tension or forces. An absorbent pad, often contained within an insert or core is then also placed into the pants-type diaper product.

To insure the pants-type diaper retains a proper shape and to hold all of the added layers of the diaper, reinforcing layers and backing materials are normally added to the continuous sheets of material, with the reinforcing layers corresponding to the cut elastic strands of each individual blank. Each of these layers needs to be adhesively joined at some point in the manufacturing process to the elastic web laminate to form the completed undergarment.

When a user soils a pants type diaper, typically the user will tear apart side seams provided on the pants type diaper in order to avoid contaminating the legs of the user. However, should the user find that the pants type diaper was not in fact soiled, the user would not have ready means for recoupling the pants type diaper about the waist for reuse.

### Summary of the Invention

The present invention provides a means for either re-fastening pants type diapers about the waist should side seams have been broken, or for providing a means for primary fastening of a pants type diaper about the waist.

The invention provides for a stretch or non-stretch type fastening tab feature (using mechanical fastener or adhesive) that can be used to refasten a disposable pant when one or more side seams have been taken apart.

This invention preferably, but not necessarily, uses an in-line process for construction instead of a transverse process which is more expensive and complex.

A refastenable feature in accordance with the present invention is preferably hidden internally in the product instead of externally. The refastenable feature is preferably hidden between material layers of the product which is more aesthetically pleasing than a refastenable feature located external on the product. This arrangement is also less likely to cause complications in the high speed machine process.

This arrangement also allows for cheaper materials to be used, and for better elastomeric properties to be used in the refastenable feature construction.

### Brief Description of the Drawings

Fig. 1 is a front view of a prior art pants type diaper.
Fig. 2 is side view of a prior art pants type diaper.
Fig. 3 is a plan view of a refastenable arrangement, in a non-expanded condition, for a pants type diaper of the present invention.
Fig. 4 is a perspective view, with portions broken away, of a refastenable arrangement, in a non-expanded condition, for a pants type diaper of the present invention.
Fig. 5 is a side perspective view of the refastenable arrangement in a non-expanded condition for a pants type diaper of the present .invention.
Fig. 6 is a side perspective view of the refastenable arrangement in an expanding condition for a pants type diaper of the present invention
Fig. 7 is a side perspective view of the refastenable arrangement in an expanded and deployed condition for a pants type diaper of the present invention.

### Description of the Preferred Embodiment

Although the disclosure hereof is detailed and exact to enable those skilled in the art to practice the invention, the physical embodiments herein disclosed merely exemplify the invention which may be embodied in other specific structures. While the preferred embodiment has been described, the details may be changed without departing from the invention.

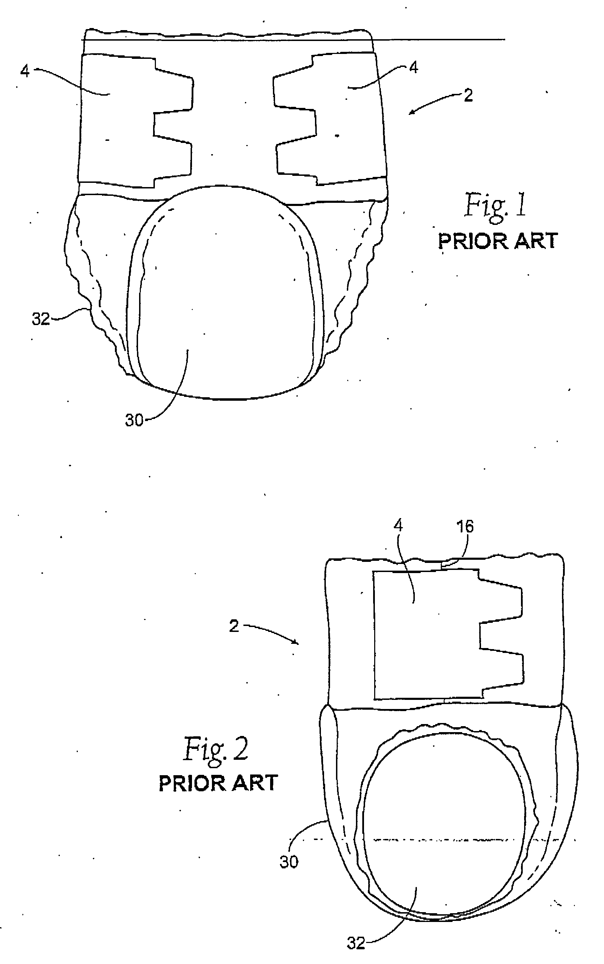

Referring now to Fig. 1, a front view of a prior art pants type diaper 2 is shown. In this view absorbent material 30 is shown and leg holes 32 are provided. Refastenable features 4 can be applied at the sides of the diaper 2.

Referring now to Fig. 2 is side view of a prior art pants type diaper is shown. Tabs 4 are provided about side seam 16. The pant product 2 has refastenable features 4 attached to the exterior of the product 2. The refastenable features 4 are used after the side-seam 16 of the pant has been torn open. This allows the consumer to use this product as a pull-up pant or as a refastenable diaper.

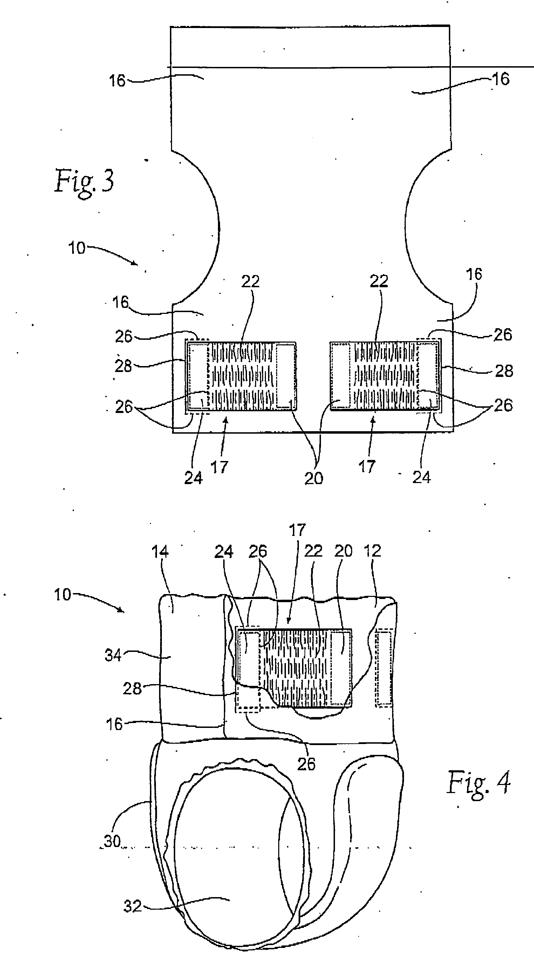

Referring now to Fig. 3 a plan view of a refastenable arrangement 17, in a non-expanded condition, for a pants type diaper 10 is shown. This invention preferably, though not necessarily, uses an in-line process for construction. As can be seen, the refastenable arrangement 17 preferably resides on either a back or a front panel of the pants type diaper 10 prior to seam 16 being formed during the manufacturing process, as the disposable garments are typically produced in a laid-flat blank format. The final form of the diaper is achieved by folding the blanks in half and sealing the blanks along the side edges 16 to form the final diaper shape. Although two fastening arrangements 17 are shown, it is understood that a single fastening arrangement 17 could be provided, or that two fastening arrangements 17 share a common anchored portion 20. Referring now to Fig. 4 a side perspective view, with portions broken away, of a refastenable arrangement 17, in a nozz-expanded condition, for a pants type diaper 10 of is shown. As can be seen, fastening element 17 is preferably sandwiched between two layers 12 and 14 of material, preferably but not limited to nonwoven material. A zone 34 can be provided with or without elasticized material to urge the waistband of the diaper 10 in compliance with the user's waist.

Fastening element 17 comprises an anchored portion 20, an elasticized portion 22, and a coupling portion 24, the use of which will be described later. Anchored portion 20 is provided to couple the fastening element 17 with one or both of layers 12 and 14. This coupling between the anchored portion 20 and one or both of layers 12 and 14 can be adhesive, mechanical or otherwise. Coupling portion 24 can likewise be adhesive, mechanical or otherwise, including, for instance, hook and loop material. Coupling portion 24 is intended for placement in the zone 34 about the waist of the user across seam 16 as will be described later.

In an alternative embodiment (not shown), the portion 22 is not elasticized, but can comprise a folded portion of material so that portion 22 can be pulled about the waist of a user. One example of this would be an accordion style portion 22 that would allow the user to withdraw portion 22 to a length beyond its initial anchor point of the coupling portion 22.

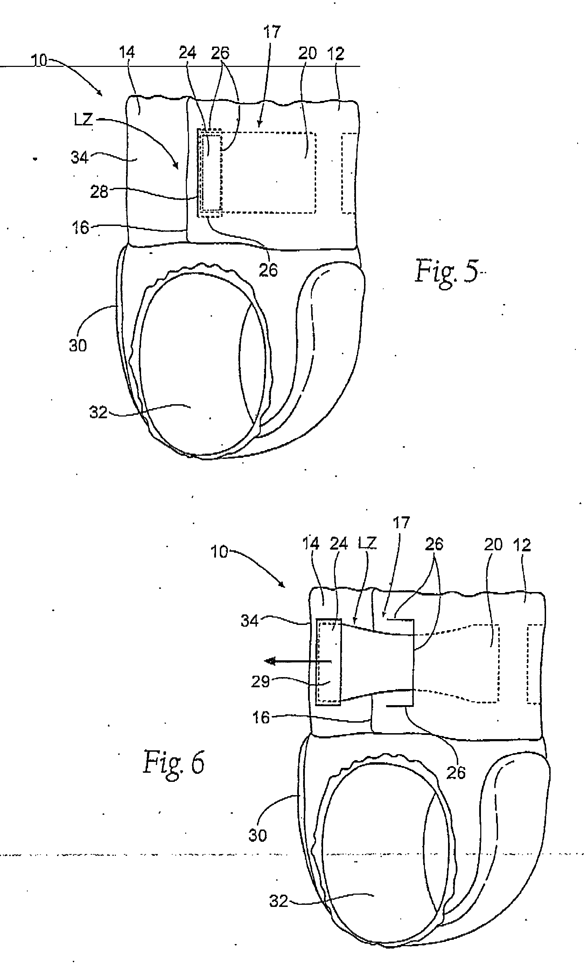

Referring now to Fig 5 is a side perspective view of the refastenable arrangement or fastening element 17 in a concealed and non-expanded condition for a pants type diaper 10 is shown. The top layer 14 of the material has perforation cuts 26 on sides of the concealed coupling portion 24. Preferably, a complete cut 28 is on one side of the coupling portion 24, said cuts 26 and 28 forming an access port to grasp removable patch 29 of the material 14 about the coupling portion 24. In this manner, the It is noted that this is merely a preferred arrangement of perforated and complete cuts 26 and 28, other arrangements or cutting types and styles are within the scope of the invention. Removable patch 29 may be coupled with coupling portion 24.

Referring now to Fig. 6 a side perspective view of the refastenable arrangement or fastening element 17 in an expanding condition for the diaper 10 of the present invention is shown. To use the present diaper 10, it is contemplated that a user will insert their finger underneath the complete cut 28 and grasp the removable patch 29 and the coupling portion 24 with another finger. Next, the user pulls on the removable patch 29 and the coupling portion 24 toward and intended landing zone LZ, to a condition which will support the diaper 10 about the waist 10 of the user. This initial pulling will free the removable patch 29 and the coupling portion 24 from the top layer 14 of the material adjacent to perforated cuts 26, which will become full incisions once the material has been freed. During pulling, elasticized portion 22 is allowed to stretch so that the fastening element 17 will provide the desired tautness about the users waist once the coupling portion 24 is applied against landing zone LZ, preferably crossing seam 16 with the coupling portion 24.

The landing zone LZ is the portion of the zone 34 that is intended to receive the coupling portion 24. As mentioned previously, because coupling portion 24 can be, for instance, comprised of hook and/or loop material, the landing zone LZ, or an area larger than the landing zone LZ, can be provided with the compliment to the hook and/or loop material. Alternatively, the zone 34 can be comprised of a material capable of receiving the hook and/or loop material. Alternatively, the landing zone LZ can be a material that is capable of receiving an adhesive from the coupling portion 24. The landing zone LZ does not have to be of a particular material or spatial configuration. Landing zone LZ is just intended to provide a coupling spot for coupling portion 24 of the fastening element 17.

Anchored portion 20 of the fastening element 17 is positioned to one side of the seam 16, the seam which may or may not be broken. Preferably, the landing zone LZ is positioned on the other side of seam 16 so that when the fastening element 17 is deployed, it will be deployed across the broken or unbroken seam 16.

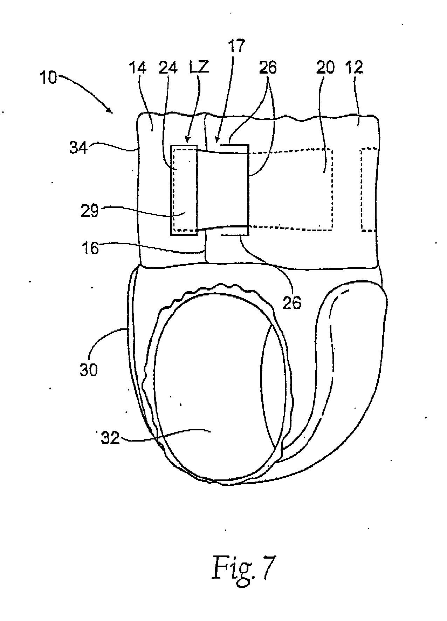

Referring now to Fig. 7 is a side perspective view of the refastenable arrangement or fastening element 17 in an expanded condition and anchored position is shown. In this view, the user has applied the stretching force to the elasticized element 22 of the fastening element by pulling on the removable patch 29 and the coupling portion 24. Next, the user has coupled removable patch 29 and the coupling portion 24 with the landing zone LZ, for instance by pressing on the removable patch 29 and the coupling portion 24.

The foregoing invention could also be deployed in a non-pants type diaper (not shown), with the fastening element 17 serving the purpose of urging front and back portions of the waistband snugly against the waist of the user.

The foregoing is considered as illustrative only of the principles of the invention. Furthermore, since numerous modifications and changes will readily occur to those skilled in the art, it is not desired to limit the invention to the exact construction and operation shown and described. While the preferred embodiment has been described, the details may be changed without departing from the invention, which is defined by the claims.

## Claims

1. A method for creating a fastening arrangement for securing an undergarment securely about the waist of a user, the method comprising:
providing an outer ply of material;
creating an access port in said outer ply;
providing a fastening element accessible through said access port, the fastening element comprising an anchor portion coupled to said outer ply, an expandable portion, an adhering portion, and a finger tab portion comprising a removable portion of said outer ply; and
providing a landing zone on said undergarment for selectively coupling said adhering portion with said landing zone.

2. The method of claim 1, wherein said access port comprises a perforated cut in said outer ply.

3. The method of claim 1, wherein said outer ply of material is a nonwoven material.

4. The method of claim 1, the method further comprising an inner ply of material, said inner ply sandwiching said fastening element.

5. A method of securing an undergarment securely about the waist of a user, the method comprising:
inserting a finger through a complete cut in an outer ply of an undergarment to thereby grasp a removable patch formed of a portion of said outer ply of said undergarment,
pulling said removable patch to stretch a stretchable portion of a fastening element;
coupling said removable patch to an area of said undergarment.
